# FASCICULE DE BREVET EUROPEEN

(11) **EP 2 881 097 B1**
(45) Date de publication et mention de la délivrance du brevet: **15.11.2017**
(21) Numéro de dépôt: 14196398.3
(22) Date de dépôt: 04.12.2014
(51) Int. Cl.: A61F 17/00, A61B 17/48

(54) **Kit d'accouchement pré-hospitalier**
Entbindungs-Set für die prähospitale Versorgung
Pre-hospital delivery kit

(30) Priorité: 06.12.2013 FR 1362246
(43) Date de publication de la demande: 10.06.2015
(73) Titulaire: Vygon, 95440 Ecouen (FR)
(72) Inventeur: Lestoquoy, Patrick, 59551 Attiches (FR); Greffe, David, 60410 Villeneuve-sur-Verberie (FR)
(74) Mandataire: Regimbeau

(56) Documents cités:
- WO-A1-01/85566
- DE-C- 168 244
- DE-C- 688 615
- FR-A- 424 280
- GB-A- 298 425
- GB-A- 496 079
- US-A- 5 848 700

## Description

### DOMAINE DE L'INVENTION

L'invention concerne d'une façon générale le matériel médical, et en l'espèce un kit d'accouchement pré-hospitalier.

Plus particulièrement, l'invention concerne un kit d'accouchement pré-hospitalier destiné aux accouchements s'effectuant dans des lieux non-adaptés.

### ARRIERE-PLAN TECHNOLOGIQUE

Un accouchement inopiné, c'est-à-dire dans un lieu non-prévisible, engendre à l'équipe médicale et à la future mère un stress supplémentaire. Ce lieu non-prévisible peut être le domicile familial, l'ambulance, le lieu de travail, la voie publique, ou tout autre lieu non dédié aux accouchements.

Lorsque cela est possible, la priorité pour l'équipe médicale est d'organiser un transport vers une maternité afin de revenir au plus vite dans un cadre sécurisé et de quitter le lieu non adapté.

Dans le cas d'un accouchement sur place, les équipes d'intervention doivent notamment s'assurer que la future mère accouchera dans les meilleures conditions possibles, et que le bébé pourra recevoir les premiers soins nécessaires.

Les documents FR 424 280, DE 688 615, GB 298 425, GB 496 079 décrivent du matériel adapté pour les accouchements mais ceux-ci ne sont pas autosuffisant pour permettre un accouchement inopiné et améliorer la qualité du service du praticien. En particulier, le document FR 424 280 divulgue des équipements permettant d'effectuer un geste opératoire sur la parturiente et/ou le nouveau-né. Ces équipements sont logés une boîte fermée. Ce document présente des lacunes pour permettre un accouchement dans de bonnes conditions (absence de champ d'accouchement par exemple, etc.)

On a ainsi proposé des kits pour accouchement inopiné, comprenant en outre du matériel stérilisé nécessaire pour permettre un accouchement dans un environnement globalement maitrisé. Néanmoins, la Demanderesse s'est aperçue du fait que, lors d'un accouchement inopiné, la gestion du stress et de l'urgence par l'équipe médicale était plus difficile que lors des accouchements dans des lieux dédiés, et que la simple présence d'un matériel médical adapté ne suffisait pas à les rassurer et à leur permettre de se concentrer sur l'assistance à la future mère et au nouveau-né.

### RESUME DE L'INVENTION

Un objectif de l'invention est donc d'améliorer les conditions de travail des équipes médicales susceptibles de participer à un accouchement inopiné, en particulier leur gestion du stress et de l'urgence, tout en garantissant à la future mère et au nouveau-né les soins nécessaires avant, pendant et après l'accouchement.

Pour cela, l'invention propose un kit d'accouchement pré-hospitalier, caractérisé en ce qu'il comprend :
- un premier ensemble destiné à une parturiente, ledit premier ensemble étant logé dans une première enveloppe et comprenant :
   * un champ d'accouchement, et
   * un premier équipement médical adapté pour effectuer un geste opératoire sur la parturiente au cours de l'accouchement,
- un deuxième ensemble destiné au nouveau-né, ledit deuxième ensemble étant logé dans une deuxième enveloppe et comprenant un deuxième équipement médical configuré pour assurer la santé immédiate du nouveau-né,
la première enveloppe et la deuxième enveloppe étant distinctes et logées dans un sachet principal fermé hermétiquement.

L'invention propose donc un kit d'accouchement pré-hospitalier, permettant une mise à disposition facile et rapide du matériel médical nécessaire que ce soit avant, pendant et/ou après l'accouchement et permet d'éviter à l'équipe médicale les risques de mélange des différents composants destinés aux différents patients (future-mère et nouveau-né), tout en permettant d'organiser simplement et complètement l'environnement de travail. Ce kit permet ainsi de diminuer le stress des opérateurs lié à l'utilisation d'un matériel d'accouchement en dehors des lieux non dédiés aux accouchements, et de les aider à se concentrer sur l'accouchement en lui-même. La parturiente et le nouveau-né sont ainsi mieux pris en main par l'équipe médicale qui diminue ses risques d'erreurs grâce à la séparation des différents ensembles.

Certaines caractéristiques préférées mais non limitatives du kit d'accouchement pré-hospitalier décrit ci-dessus sont les suivantes :
- la première enveloppe est formée par le champ d'accouchement, qui enveloppe le premier équipement médical,
- le kit d'accouchement comprend en outre des gants stériles, lesdits gants stériles étant de préférence logés dans une troisième enveloppe,
- la deuxième enveloppe est stérile et fermée hermétiquement,
- le premier équipement médical du premier ensemble destiné à la parturiente comprend l'un au moins des éléments suivants : une casaque pour protéger l'opérateur, un champ de table absorbant, un champ d'intimité pour couvrir tout ou partie de la parturiente, un sac, de préférence refermable, une compresse, de préférence plusieurs compresses, une pince adaptée pour rompre la poche des eaux, une paire de ciseaux, adaptée pour effectuer une épisiotomie, un scalpel, au moins un tampon d'épisiotomie, au moins un pansement adapté pour comprimer une hémorragie, un plateau, pour recueillir un placenta, une sonde urinaire une pince occlusive, dite clamp,
- le deuxième équipement médical du deuxième ensemble destiné au nouveau-né comprend l'un au moins des éléments suivants : une serviette absorbante pour sécher le nouveau-né, un jersey configuré pour réaliser un bonnet, un champ nouveau-né adapté pour couvrir tout ou partie du nouveau-né, un aspirateur de mucosité, pour libérer les voies aériennes du nouveau-né, au moins une pince occlusive, une couverture pour limiter les pertes de chaleur du nouveau-né.
- le kit d'accouchement comprend en outre un champ absorbant jaugé muni d'une étiquette inscriptible, et
- le champ d'accouchement comprend des poches collectrices de fluides.

L'invention propose également un champ d'accouchement jaugé, adapté pour être utilisé dans un kit pour accouchement pré-hospitalier comme décrit ci-dessus, ledit champ d'accouchement comprenant une feuille principale réalisée dans un matériau absorbant et étant caractérisé en ce qu'il comprend une zone inscriptible.

Dans une forme de réalisation, la zone inscriptible comprend une étiquette.

### BREVE DESCRIPTION DES DESSINS

D'autres caractéristiques, buts et avantages de la présente invention apparaîtront mieux à la lecture de la description détaillée qui va suivre, et au regard des dessins annexés donnés à titre d'exemples non limitatifs et sur lesquels :

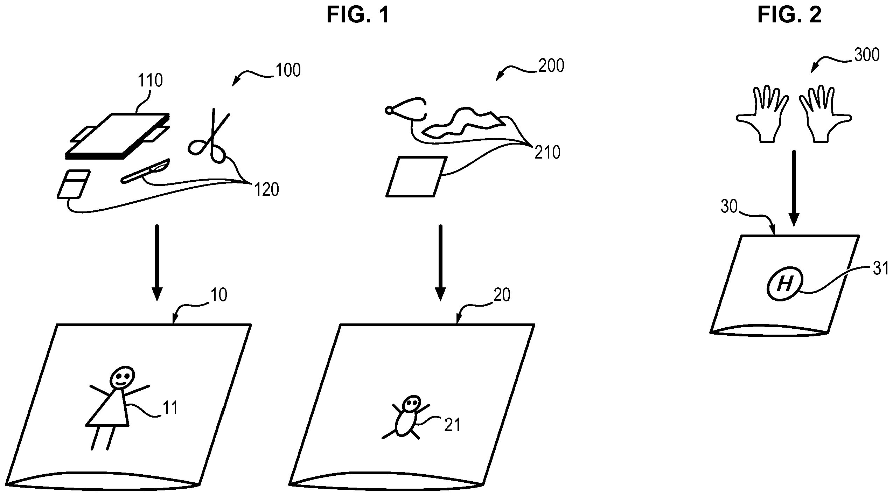

La figure 1 représente un exemple de réalisation d'un kit d'accouchement pré-hospitalier conforme à l'invention.
La figure 2 représente une paire de gants et son enveloppe.

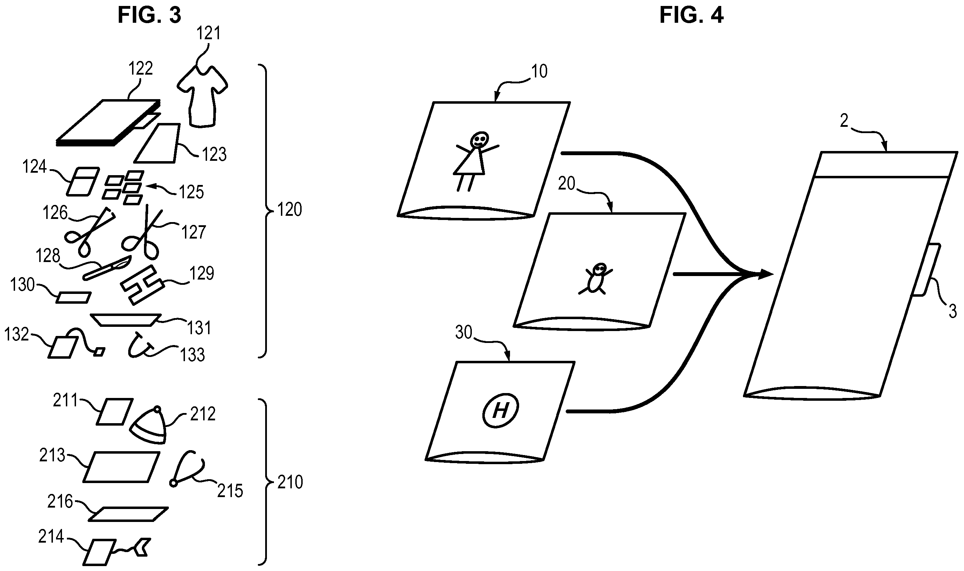

La figure 3 représente un exemple de réalisation d'un équipement médical susceptible d'être utilisé dans un kit d'accouchement pré-hospitalier conforme à l'invention.
La figure 4 illustre l'agencement général du kit d'accouchement pré-hospitalier tel que défini dans l'invention.
La figure 5 représente un exemple de réalisation d'un champ d'accouchement susceptible d'être utilisé dans un kit d'accouchement pré-hospitalier conforme à l'invention.
La figure 6 illustre le déploiement d'une première variante de réalisation d'une première enveloppe d'un kit d'accouchement selon l'invention, et
La figure 7 schématise un procédé d'utilisation de l'invention.

### DESCRIPTION DETAILLEE D'UN MODE DE REALISATION

Nous allons à présent décrire un kit d'accouchement inopiné conforme à la présente invention.

Par accouchement, nous désignons toute la période comprenant la perte des eaux, la délivrance (extraction du placenta), le suivi des pertes sanguines et la prise en charge effective du nouveau-né.

Par parturiente, nous désignons la personne sur le point d'accoucher et qui vient d'accoucher : la parturiente peut donc désigner la femme enceinte sur le point d'accoucher ou la nouvelle mère.

Par geste opératoire, nous entendons tout geste ou acte effectué par l'opérateur concourant au bon déroulement de l'accouchement.

Un kit conforme à l'invention comprend un premier ensemble 100 destiné à une parturiente, et un deuxième ensemble 200 destiné au nouveau-né.

Chacun des ensembles 100, 200 est logé dans une enveloppe 10, 20 respectives et distinctes. Les deux enveloppes 10, 20 contenant les ensembles sont elles-mêmes logés dans un sachet principal 2 fermé hermétiquement. Par exemple, le sachet principal 2 peut être réalisé dans un matériau plastique thermosoudé qu'il suffit de déchirer ou de peler afin d'accéder à son contenu. Un tel sachet principal 2 peut alors avantageusement être stérilisé et maintenu stérile.

Le premier ensemble 100 comprend un champ d'accouchement 110, et un équipement médical 120 adapté pour effectuer un geste opératoire sur la parturiente au cours de l'accouchement.

Le deuxième ensemble 200 comprend un équipement médical 210 configuré pour assurer la santé immédiate du nouveau-né.

En séparant ainsi les équipements destinés à la parturiente et au nouveau-né, la gestion de l'urgence et du stress de l'opérateur sont nettement améliorés. L'opérateur peut en effet, dès le début de l'accouchement, saisir uniquement l'ensemble 100 destiné à la parturiente, sans se préoccuper de l'ensemble 200 destiné au nouveau-né, celui-ci n'étant pas encore présent.

Dans une forme de réalisation, le premier ensemble 100 peut comprendre un champ d'accouchement 110 et au moins un équipement médical 120 adapté pour effectuer un geste opératoire sur la parturiente au cours de l'accouchement. Par exemple, l'équipement médical 120 du premier ensemble 100 peut comprendre au moins l'un parmi les éléments suivants :
- une casaque 121, pour protéger l'opérateur,
- un champ absorbant 122, réalisé dans un matériau susceptible d'absorber les fluides (flux physiologiques, pertes sanguines, etc.), du type Absorbex®,
- un champ d'intimité 123, configuré pour couvrir tout ou partie de la parturiente, par exemple son buste,
- un sac 124, pouvant être refermable, par exemple du type sacs à déchets ou des sacs DASRI (Déchets d'Activités de Soins à Risques Infectieux et assimilés), configuré pour recueillir les différents déchets issus de l'accouchement et des opérations associées,
- une compresse 125, de préférence plusieurs,
- une pince 126 adaptée pour rompre la poche des eaux, par exemple une pince du type Kocher,
- une paire de ciseaux 127 configurée pour réaliser une épisiotomie, par exemple des ciseaux courbes Metzenbaum,
- un scalpel 128, pouvant notamment être utilisé pour couper le cordon ombilical,
- un tampon d'épisiotomie 129,
- un pansement 130 adapté pour stopper des hémorragies, par exemple un pansement américain,
- un plateau 131, pour recevoir le placenta,
- une sonde urinaire 132,
- une pince occlusive 133, dite « clamp ».

Le champ d'accouchement 110 et le ou les premiers équipements médicaux 120 formant l'ensemble 100 destiné à la parturiente sont logés dans la première enveloppe 10.

Selon une première variante de réalisation, la première enveloppe 10 peut être formée par le champ d'accouchement 110 lui-même. Le champ d'accouchement 10 est alors plié de manière à contenir le ou les premiers équipements médicaux 120.

Selon une deuxième variante de réalisation, la première enveloppe 10 peut être un sachet réalisé dans un matériau plastique thermosoudé, qu'il suffit de déchirer afin d'accéder à son contenu (à savoir le champ d'accouchement 110 et le ou les premiers équipements 120). La première enveloppe 10 est alors de préférence stérile et fermée hermétiquement.

Le ou les premiers équipements médicaux 120 du premier ensemble 100 sont de préférence agencés de façon à optimiser le volume de la première enveloppe 10.

Afin de faciliter la manipulation du kit d'accouchement 1, le premier ensemble 100 peut comprendre des moyens d'identification 11, permettant à l'opérateur d'identifier rapidement le premier ensemble 100, limitant ainsi les erreurs possibles, ce qui concourt à diminuer le stress des opérateurs et à gagner un maximum de temps pour la mise en place du kit d'accouchement. Les moyens d'identification 11 peuvent notamment comprendre une inscription sur la première enveloppe 10 (dénomination verbale, illustrations, pictogrammes, etc.) ou un jeu de couleur spécifique.

De manière conventionnelle, le champ d'accouchement 110 peut être du type champ de table, et comprendre éventuellement une ou plusieurs poches de recueil, adaptées pour recueillir les fluides physiologiques et/ou les pertes sanguines.

En variante, la mesure des fluides physiologiques et/ou des pertes sanguines peut être réalisée avec des champs absorbants spécifiques, tels que des champs absorbants réalisés dans un matériau du type Absorbex®.

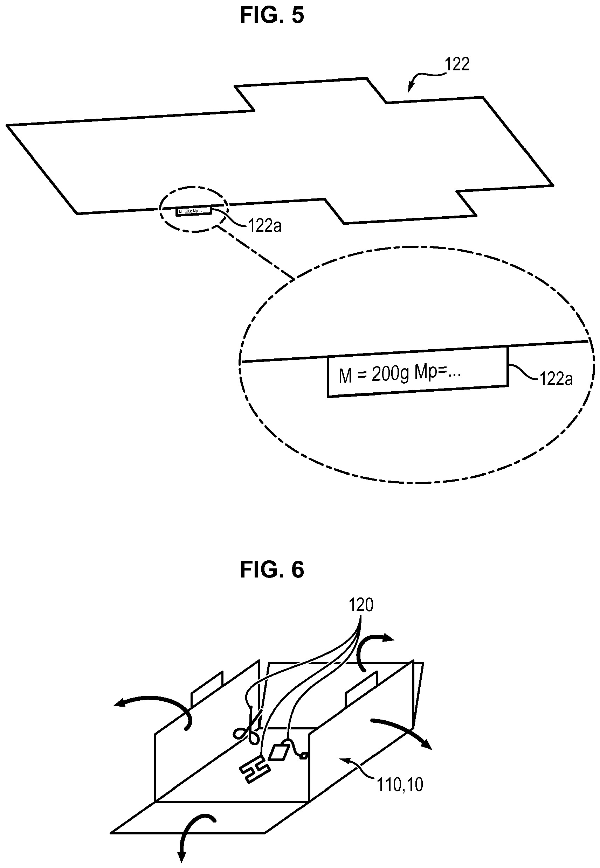

Un exemple de réalisation d'un tel champ absorbant 122 permettant de mesurer les pertes de fluides physiologiques et/ou les pertes sanguines a été illustré en figure 5. Un tel champ absorbant 122 est configuré pour absorber l'intégralité des fluides physiologiques et/ou des pertes sanguines, le volume de fluides perdu par la parturiente étant ensuite mesuré en déterminant le poids du champ absorbant 122 et en soustrayant à ce poids le poids initial du champ absorbant.

Afin de faciliter les mesures, le champ absorbant 122 est jaugé préalablement à son utilisation. Par jaugé, on comprendra ici que le poids initial du champ absorbant est déterminé. Ce poids initial peut ensuite être inscrit sur le champ absorbant 122 dans une zone inscriptible 122a dudit champ 122, par exemple sur une étiquette 122a fixée sur le champ absorbant 122.

Dans une forme de réalisation, le champ absorbant 122 est jaugé préalablement à son introduction dans la première enveloppe 10. En variante, le champ absorbant 122 peut être jaugé directement par l'opérateur, suite à l'ouverture de la première enveloppe 10.

Une fois que la parturiente a perdu l'ensemble des fluides que l'opérateur cherche à mesurer (fluides physiologiques ou ultérieurement pertes sanguines), l'opérateur peut alors retirer le champ absorbant jaugé 122 et le remplacer par un nouveau champ absorbant afin de remettre la parturiente au propre.

Le poids du champ absorbant jaugé 122 et contenant les fluides peut alors être déterminé, sur place ou ultérieurement, afin d'évaluer la perte de fluides (fluides physiologiques ou pertes sanguines).

Dans l'exemple de réalisation illustré sur la figure 5, le champ absorbant jaugé 122 comprend une étiquette 122a sur laquelle a été inscrit, préalablement à l'introduction du champ 122 dans la première enveloppe 10, le poids initial « à vide » du champ absorbant 122. Ce champ absorbant 122 peut alors être utilisé soit pour la mesure des fluides physiologiques, soit pour la mesure des pertes sanguines. De préférence, le premier ensemble 100 comprend un champ absorbant jaugé 122 par type de fluides dont on souhaite suivre le volume. Une fois l'ensemble des fluides absorbés par le champ absorbant jaugé 122, l'opérateur peut alors déterminer son poids, puis l'inscrire sur l'étiquette 122a. Alternativement, ce poids peut être inscrit sur une autre étiquette présente sur le champ absorbant jaugé 122. Suite à l'accouchement, il sera alors possible de déterminer le volume de fluides perdus par la parturiente, et le cas échéant de réaliser les opérations nécessaires. Par exemple, le poids du champ absorbant jaugé 122 peut être déterminé par un personnel hospitalier.

On comprendra qu'un tel champ absorbant jaugé 122 remplace de manière simple les poches de recueil, en permettant à la fois de recueillir et de mesurer le niveau des pertes de fluides (fluides physiologiques ou pertes sanguines), quelles que soient les circonstances, la position de la parturiente, etc. En particulier, il n'est plus nécessaire de placer la parturiente de manière à garantir que les fluides s'écoulent dans une poche de recueil, qui par définition ne peut être positionnée horizontalement.

L'étiquette 122a est de préférence inscriptible, et peut le cas échéant être protégée du contact de l'extérieur pour limiter les risques de détérioration.

Par ailleurs, on comprendra en outre que l'utilisation d'un tel champ absorbant jaugé 122 n'est pas limitée à un accouchement inopiné, et qu'il peut également être utilisé, en remplacement ou en sus d'une poche de recueil, lors d'un accouchement dans des conditions maîtrisées et indépendamment de l'utilisation ou non d'un kit pour accouchement pré-hospitalier tel que celui décrit dans cette invention.

Le deuxième ensemble 200 destiné au nouveau-né comprend au moins un équipement 210 adapté pour assurer la santé immédiate du nouveau-né. Par exemple, le deuxième équipement médical 210 du deuxième ensemble 200 peut comprendre au moins l'un parmi les éléments suivants :
- une serviette absorbante 211 pour sécher le nouveau-né et limiter en particulier l'hypothermie,
- un jersey 212, pour réaliser un bonnet et limiter l'hypothermie,
- un champ nouveau-né 213 pour couvrir le nouveau-né,
- un aspirateur de mucosité 214, pour libérer les voies aériennes si nécessaire,
- une ou plusieurs pinces occlusives 215,
- une couverture adaptée 216 pour limiter les pertes de chaleur pour éviter l'hypothermie du nouveau-né, telle qu'une couverture de survie ou tout autre champ spécialisé tel que les Calor keeper.

De manière analogue au sachet principal 2, la deuxième enveloppe 20 peut être stérile et fermé hermétiquement. Par exemple, la deuxième enveloppe 20 peut être un sachet réalisé dans un matériau plastique thermosoudé, qu'il suffit de déchirer ou peler afin d'accéder à son contenu. Par ailleurs, la deuxième enveloppe 20 peut également comprendre des moyens d'identification 21 (inscription sur l'enveloppe, un jeu de couleur spécifique, etc.), permettant à l'opérateur d'identifier rapidement le deuxième ensemble 200, limitant ainsi les erreurs possibles.

Le ou les deuxièmes équipements médicaux du deuxième ensemble 200 sont de préférence agencés de façon à optimiser le volume de la deuxième enveloppe 20.

Dans une forme de réalisation, le kit d'accouchement pré-hospitalier 1 peut en outre comprendre au moins une paire de gants stériles 300, de préférence deux paires, pour permettre à l'opérateur de se protéger et de protéger la parturiente lors de l'accouchement.

Les gants stériles 300 peuvent être logés dans une troisième enveloppe 30 de protection, qui peut être stérile et fermée hermétiquement. Par exemple, la troisième enveloppe 30 peut être un sachet réalisé dans un matériau plastique thermosoudé, qu'il suffit de déchirer ou peler afin d'accéder à son contenu. La troisième enveloppe 30 peut également comprendre des moyens d'identification 31 (inscription sur l'enveloppe, un jeu de couleur spécifique, etc.), permettant à l'opérateur d'identifier rapidement son contenu (gants stériles 300), limitant ainsi les erreurs possibles.

La troisième enveloppe contenant les gants stériles 300 peut être placée soit dans la première enveloppe 10, ou en variante en dehors de la première enveloppe 10 et de la deuxième enveloppe 20, c'est-à-dire directement dans le sachet principal 2.

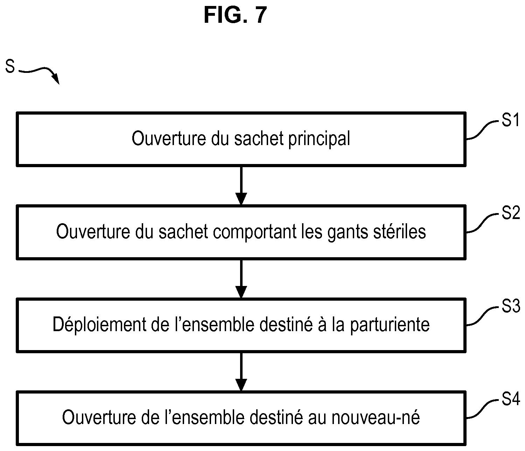

Nous allons à présent décrire un exemple de procédé d'utilisation S d'un kit d'accouchement 1 (cf. figure 7). Dans cet exemple, le kit d'accouchement 1 comprend le premier ensemble 100, le deuxième 200 ensemble et des gants stériles 300, logés respectivement dans une première enveloppe 10, une deuxième enveloppe 20 et un troisième 30 enveloppe. Par ailleurs, le premier ensemble 100 comprend au moins un champ absorbant jaugé 122. Ceci n'est cependant pas limitatif, dans la mesure où les gants stériles 300 et le champ absorbant jaugé 122 sont optionnels.

Dans un premier temps S1, l'opérateur ouvre le sachet principal 2, par exemple en le déchirant ou en le pelant. L'opérateur saisit la troisième enveloppe 30 comportant les gants stériles 300, l'ouvre (étape S2) et enfile les gants 300.

Dans un deuxième temps S3, l'opérateur peut déployer l'ensemble 100 (soit en dépliant le champ d'accouchement 110, qui forme la première enveloppe 10, soit en ouvrant la première enveloppe 10 directement, selon la variante de réalisation de l'ensemble 100) afin de prendre le champ d'accouchement 110 et le positionner sur une surface de travail pour mettre cette surface au propre en vue de l'accouchement. La parturiente peut alors être installée sur le champ opératoire.

La deuxième enveloppe 20 reste en revanche fermée, afin d'éviter à l'opérateur de mélanger son contenu 200 avec celui du premier ensemble 100.

L'accouchement peut alors avoir lieu. Afin de réaliser cet accouchement dans les meilleures conditions possibles, l'opérateur peut utiliser l'ensemble des premiers équipements médicaux présents dans le premier ensemble 100.

En particulier, l'opérateur peut positionner un champ absorbant jaugé 122 par-dessus le champ d'accouchement 110 et sous les fesses de la parturiente afin d'absorber le maximum de fluides (fluides physiologiques ou pertes sanguines, selon le moment de l'accouchement) et permettre leur mesure ultérieure par soustraction du poids mesuré avec le poids initial inscrit sur l'étiquette 122a.

Après la naissance du nouveau-né, l'opérateur peut ouvrir la deuxième enveloppe 20 afin de prendre le ou les deuxièmes équipements nécessaires 210 pour assurer la santé immédiate du nouveau-né (étape S4), et notamment effectuer les gestes nécessaires pour permettre au nouveau-né notamment de ne pas souffrir d'hypothermie.

De par la séparation des différents éléments le constituant en deux ensembles 100, 200 séparés et distincts, le kit d'accouchement 1 de l'invention permet donc à l'opérateur de rationaliser l'accouchement, malgré les circonstances, et de simplifier ses gestes et actes tout au long de l'accouchement, tout en garantissant à la future mère et au nouveau-né les soins nécessaires avant, pendant et après l'accouchement.

## Revendications

1. Kit d'accouchement (1) pré-hospitalier, **caractérisé en ce qu'**il comprend :
- un premier ensemble (100) destiné à une parturiente, ledit premier ensemble (100) étant logé dans une première enveloppe (10) et comprenant :
* un champ d'accouchement (110), et
un premier équipement médical (120) adapté pour effectuer un geste opératoire sur la parturiente au cours de l'accouchement,
- un deuxième ensemble (200) destiné au nouveau-né, ledit deuxième ensemble (200) étant logé dans une deuxième enveloppe (20) et comprenant un deuxième équipement médical (210) configuré pour assurer la santé immédiate du nouveau-né,
la première enveloppe (10) et la deuxième enveloppe (20) étant distinctes et logées dans un sachet principal (2) fermé hermétiquement.

2. Kit d'accouchement (1) selon la revendication 1, dans lequel la première enveloppe (10) est formée par le champ d'accouchement (110), qui enveloppe le premier équipement médical (120).

3. Kit d'accouchement (1) selon l'une des revendications 1 ou 2 comprenant en outre des gants stériles (300), lesdits gants stériles étant de préférence logés dans une troisième enveloppe (30).

4. Kit d'accouchement (1) selon l'une des revendications 1 à 3, dans lequel la deuxième enveloppe (20) est stérile et fermée hermétiquement.

5. Kit d'accouchement (1) selon l'une des revendications 1 à 4, dans lequel le premier équipement médical (120) du premier ensemble (100) destiné à la parturiente comprend l'un au moins des éléments suivants :
- une casaque (121), pour protéger l'opérateur,
- un champ de table absorbant (122),
- un champ d'intimité pour couvrir tout ou partie de la parturiente (123),
- un sac, de préférence refermable (124),
- une compresse, de préférence plusieurs compresses (125),
- une pince adaptée pour rompre la poche des eaux (126),
- une paire de ciseaux (127), adaptée pour effectuer une épisiotomie,
- un scalpel (128),
- au moins un tampon d'épisiotomie (129),
- au moins un pansement adapté pour comprimer une hémorragie (130),
- un plateau (131), pour recueillir un placenta,
- une sonde urinaire (132)
- une pince occlusive, dite clamp (133).

6. Kit d'accouchement (1) selon l'une des revendications 1 à 5, dans lequel le deuxième équipement médical (210) du deuxième ensemble (200) destiné au nouveau-né comprend l'un au moins des éléments ci-dessous :
- une serviette absorbante pour sécher le nouveau-né (211),
- un jersey configuré pour réaliser un bonnet (212),
- un champ nouveau-né (213) adapté pour couvrir tout ou partie du nouveau-né,
- un aspirateur de mucosité (214), pour libérer les voies aériennes du nouveau-né,
- au moins une pince occlusive (215),
- une couverture (216) pour limiter les pertes de chaleur du nouveau-né.

7. Kit d'accouchement (1) selon l'une des revendications 1 à 6, comprenant en outre un champ absorbant jaugé (122) muni d'une étiquette inscriptible (122a).

8. Kit d'accouchement (1) selon l'une des revendications 1 à 7, dans lequel le champ d'accouchement (110) comprend des poches collectrices de fluides.

## Patentansprüche

1. Entbindungskit (1) für prähospitale Versorgung, **dadurch gekennzeichnet, dass** es umfasst:
- eine erste Einheit (100), die für eine Gebärende bestimmt ist, wobei die erste Einheit (100) in einer ersten Hülle (10) untergebracht ist und umfasst:
* ein Entbindungstuch (110), und
- eine erste medizinische Ausrüstung (120), die ausgebildet ist, um an der Gebärenden während der Entbindung eine operative Geste durchzuführen,
- eine zweite Einheit (200), die für das Neugeborene bestimmt ist, wobei die zweite Einheit (200) in einer zweiten Hülle (20) untergebracht ist und eine zweite medizinische Ausrüstung (210) umfasst, die konfiguriert ist, um sofort die Gesundheit des Neugeborenen zu sichern,
wobei die erste Hülle (10) und die zweite Hülle (20) unterschiedlich sind und in einem hermetisch verschlossenen Hauptbeutel (2) untergebracht sind.

2. Entbindungskit (1) nach Anspruch 1, wobei die erste Hülle (10) von dem Entbindungstuch (110) gebildet ist, welches die erste medizinische Ausrüstung (120) umhüllt.

3. Entbindungskit (1) nach einem der Ansprüche 1 oder 2, umfassend ferner sterile Handschuhe (300), wobei die sterilen Handschuhe vorzugsweise in einer dritten Hülle (30) untergebracht sind.

4. Entbindungskit (1) nach einem der Ansprüche 1 bis 3, wobei die zweite Hülle (20) steril und hermetisch verschlossen ist.

5. Entbindungskit (1) nach einem der Ansprüche 1 bis 4, wobei die erste medizinische Ausrüstung (120) der ersten Einheit (100), die für die Gebärende bestimmt ist, mindestens eines der folgenden Elemente umfasst:
- ein Hemd (121), um den Operateur zu schützen,
- ein absorbierendes Tischtuch (122),
- ein Intimitätstuch, um die Gebärende (123) ganz oder teilweise zu bedecken,
- eine vorzugsweise wiederverschließbare Tasche (124),
- eine Kompresse, vorzugsweise mehrere Kompressen (125),
- eine Zangenschere, die geeignet ist, die Fruchtblase (126) zu öffnen,
- eine Schere (127), die geeignet ist, eine Episiotomie durchzuführen,
- ein Skalpell (128),
- mindestens einen Episiotomietupfer (129),
- mindestens einen Verband zum Komprimieren einer Hämorrhagie (130),
- ein Tablett (131) zur Aufnahme einer Plazenta,
- eine Urinsonde (132),
- eine Verschlussklemme, als Clamp (133) bezeichnet.

6. Entbindungskit (1) nach einem der Ansprüche 1 bis 5, wobei die zweite medizinische Ausrüstung (210) der zweiten Einheit (200), die für das Neugeborene bestimmt ist, mindestens eines der folgenden Elemente umfasst:
- ein saugfähiges Handtuch, um das Neugeborene (211) abzutrocknen,
- ein Jersey, das konfiguriert ist, um eine Mütze (212) zu bilden,
- ein Neugeborenentuch (213), das geeignet ist, das Neugeborene ganz oder teilweise abzudecken,
- einen Schleimabsauger (214) zur Befreiung der Luftwege des Neugeborenen,
- mindestens eine Verschlussklemme (215),
- eine Decke (216), um die Wärmeverluste des Neugeborenen zu begrenzen.

7. Entbindungskit (1) nach einem der Ansprüche 1 bis 6, umfassend ferner ein charakterisiertes saugfähiges Tuch (122) mit einem beschriftbaren Etikett (122a).

8. Entbindungskit (1) nach einem der Ansprüche 1 bis 7, wobei das Entbindungstuch (110) Fluidsammeltaschen umfasst.

## Claims

1. A pre-hospital delivery kit (1), **characterized in that** it comprises:
- a first assembly (100) for a parturient woman, said first assembly (100) being housed in a first envelope (10) and comprising:
* a delivery drape (110), and
- a first medical equipment (120) suitable for performing an operative procedure on the parturient woman during delivery,
- a second assembly (200) for the newborn, said second assembly (200) being housed in a second envelope (20) and comprising a second medical equipment (210) configured to ensure the immediate health of the newborn,
the first envelope (10) and the second envelope (20) being distinct and housed in a main pack (2) hermetically closed.

2. The delivery kit (1) according to claim 1, wherein the first envelope (10) is formed by the delivery drape (110) which envelops the first medical equipment (120).

3. The deliver kit (1) according to one of claims 1 or 2, further comprising sterile gloves (300), said sterile gloves preferably being housed in a third envelope (30).

4. The delivery kit (1) according to one of claims 1 to 3, wherein the second envelope (20) is sterile and hermetically closed.

5. The delivery kit (1) according to one of claims 1 to 4, wherein the first medical equipment (120) of the first assembly (100) for the parturient woman comprises at least one of the following elements:
- a blouse (121) for protecting the operator,
- an absorbent table drape (122),
- a privacy drape for covering all or part of the parturient women (123),
- a preferably re-closable bag (124),
- a compress, preferably several compresses (125),
- a forceps suitable for breaking the bag of water (126),
- a pair of scissors (127), suitable for performing an episiotomy,
- a scalpel (128),
- at least one episiotomy tampon (126),
- at least one bandage suitable for compressing a hemorrhage (130),
- a plate (131) for collecting a placenta,
- a urinary catheter (132),
- an occlusive clamp, called a clamp (133).

6. The delivery kit (1) according to one of claims 1 to 5, wherein the second medical equipment (210) of the second assembly (200) for the newborn comprises at least one of the elements below:
- an absorbent towel for drying the newborn (211),
- a jersey configured for creating a bonnet (212),
- a newborn drape (213) suitable for covering all or part of the newborn,
- a mucus aspirator (214) to clear the airways of the newborn,
- at least one occlusive clamp (215),
- a blanket (216) for limiting heat losses from the newborn.

7. The delivery kit (1) according to one of claims 1 to 6, further comprising a graduated absorbent drape (122) equipped with a writeable label (122a).

8. The delivery kit (1) according to one of claims 1 to 7, wherein the delivery drape (110) comprises fluid-collecting pockets.
